(19) Europäisches Patentamt
European Patent Office
Office européen des brevets

(11) Numéro de publication: **0 087 329**
**B1**

(12) # FASCICULE DE BREVET EUROPEEN

(45) Date de publication du fascicule du brevet: **16.10.85**

(51) Int. Cl.[4]: **A 61 L 15/07**, C 08 L 67/04

(21) Numéro de dépôt: **83400143.0**

(22) Date de dépôt: **20.01.83**

(54) **Procédé de préparation d'une nouvelle composition thermoplastique à mémoire à partir de polycaprolactone et de polyuréthane, produit obtenu selon ce procédé et son utilisation notamment en orthopédie.**

(30) Priorité: **20.01.82 FR 8200854**

(43) Date de publication de la demande:
**31.08.83 Bulletin 83/35**

(45) Mention de la délivrance du brevet:
***16.10.85 Bulletin 85/42***

(84) Etats contractants désignés:
**AT BE CH DE FR GB IT LI LU NL SE**

(56) Documents cités:
**FR - A - 2 178 726**
**FR - A - 2 369 830**
**GB - A - 1 408 142**
**US - A - 3 832 333**

(73) Titulaire: **LABORATOIRES D'HYGIENE ET DE DIETETIQUE L.H.D. Société Anonyme dite:, 38 avenue Hoche, F-75008 Paris (FR)**

(72) Inventeur: **Grouiller, Hervé, 1, rue d'Aval à Chevrey, F-21700 Arcenant (FR)**

(74) Mandataire: **Combe, André, CABINET BEAU DE LOMENIE 55 rue d'Amsterdam, F-75008 Paris (FR)**

**EP 0 087 329 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation d'une nouvelle composition thermoplastique à mémoire à partir de polycaprolactone et de polyuréthanne. Elle concerne aussi en tant que produit industriel nouveau, la composition thermoplastique obtenue selon ce procédé. Elle concerne également l'utilisation de ce produit industriel nouveau notamment en orthopédie en tant que moyen de contention.

On sait que la matière la plus utilisée pour immobiliser une partie du corps humain, et notamment un membre fracturé, est le plâtre. Il se trouve que, dans le domaine des structures orthopédiques, les bandages au plâtre constituent un moyen de contention présentant un grand nombre d'inconvénients. En effet, le plâtre est lourd et peu perméable à l'air, il absorbe et disperse les rayons X, ce qui gêne les diagnostics par radiographie, et il donne souvent lieu à des irritations de la peau.

On sait par ailleurs que, pour remplacer le plâtre, on a proposé dans le passé d'utiliser des structures orthopédiques en polycaprolactone. La polycaprolactone est une substance qui présente à la température ambiante une grande résistance à la flexion et un module d'élasticité élevé, mais qui a l'inconvénient à partir de 50° C de devenir molle et très collante. Si on chauffe la polycaprolactone en feuille à une température supérieure à environ 50° C, elle perd rapidement sa résistance mécanique et son élasticité, et elle ne peut même pas supporter son propre poids.

On vient de trouver de façon surprenante que, à partir de polycaprolactone (en abrégé PCL) et de polyuréthanne (en abrégé PU), on obtient selon les modalités opératoires définies ci-après, une composition thermoplastique nouvelle constituant un produit industriel doué de mémoire élastique et qui présente des propriétés particulièrement intéressantes en ce qui concerne la résistance mécanique à la chaleur. Ce produit qui est notamment utile en tant que moyen orthopédique de contention, (i) est, par rapport au plâtre, d'un usage plus facile et d'un poids plus léger, et (ii) ne perd pas totalement, par rapport à la PCL, sa résistance mécanique, notamment à 60° C.

La présente invention concerne donc un procédé de préparation de mélanges de matières plastiques dont les réseaux sont imbriqués, lesdites matières plastiques étant, au stade final du mélange une polycaprolactone et au moins un polyuréthanne, ledit procédé étant caractérisé en ce qu'on réalise la synthèse d'un polyuréthanne, à partir des éléments connus de préparation dudit polyuréthanne à savoir au moins un polyol et au moins un polyisocyanate, au sein d'une polycaprolactone.

Le simple mélange de la PCL avec le PU préalablement polymérisé ne convenant pas du point de vue des propriétés mécaniques, il est très important, selon l'invention, que la formation du polyuréthanne à partir de polyol et de polyisocyanate soit effectuée ou se poursuive dans la polycaprolactone à l'état ramolli, de façon que la polymérisation du PU soit réalisée avec imbrication du réseau du PU faiblement thermoplastique ou non thermoplastique, dans le réseau fortement thermoplastique de la PCL. En bref, on pourra dans ce but mettre en œuvre deux variantes A et B, la variante A étant la variante préférée:

– Variante A. Mélanger la PCL avec les moyens formant le PU, à savoir les polyols et les polyisocyanates, puis procéder à la réaction de polymérisation du PU;

– Variante B. Initier la réaction de polymérisation du PU à partir des moyens le constituant, mélanger le milieu réactionnel résultant avec la PCL de façon que la polymérisation du PU se poursuive dans la PCL. Selon un mode avantageux de mise en œuvre du procédé de l'invention, la formation du PU est réalisée selon les modalités opératoires données ci-dessus, à partir d'un moyen I choisi parmi l'ensemble constitué par les polyisocyanates et leurs mélanges, et d'un moyen II choisi parmi l'ensemble constitué par les polyols et leurs mélanges, les moyens I et II étant tels que, avant réaction de polymérisation, le nombre de groupes NCO libres du moyen I soit sensiblement égal au nombre de groupes OH libres du moyen II.

De façon avantageuse, le produit thermoplastique selon l'invention sera préparé par réaction des moyens I et II dans la PCL, de telle façon que l'on obtienne une composition finale comprenant:

A) 60 à 100 parties en poids de PCL, et

B) 2 à 40 parties en poids de PU.

Le cas échéant, la formation du PU dans la PCL pourra être effectuée en présence d'un ou plusieurs adjuvants, notamment au moins un moyen choisi parmi l'ensemble constitué par les charges minérales, les colorants et les plastifiants. On pourra ainsi utiliser pour 60 à 100 parties en poids de PCL

C) au plus 35 parties en poids de charge minérale, et/ou

D) au plus 5 parties en poids d'agent plastifiant.

Les quantités préférées sont de 5 à 35 parties en poids pour le moyen C et de 1 à 2 parties en poids pour le moyen D, quand, bien entendu, lesdits moyens C et/ou D sont utilisés. Parmi les charges minérales qui conviennent, on peut notamment mentionner ZnO, $CaCO_3$, $TiO_2$, $Ta_2O_5$ et le talc; et parmi les plastifiants qui conviennent, on peut notamment mentionner l'acide stéarique qui joue également le rôle d'agent lubrifiant.

Le meilleur mode de mise en œuvre du procédé de l'invention comprend:

1. le mélange sous agitation de la PCL avec les moyens I et II, à une température comprise entre 75 et 130° C, pendant 1 à 10 min, pour initier la formation du PU dans le réseau de la PCL, puis

2. la poursuite de la formation du PU dans le réseau de la PCL à une température supérieure ou égale à 60° C (et de préférence comprise entre 60 et 100° C), pendant 10 à 30 min.

Les moyens C et/ou D, quand ils seront présents, seront incorporés au stade 1°.

De façon avantageuse, on préconise au stade 1° d'effectuer un malaxage dans un malaxeur étanche, à 50-300 tr/min, et de préférence à

150 tr/min. Le malaxage peut être effectué sous atmosphère d'azote notamment quand il est réalisé à une température comprise entre 100 et 130° C. De préférence, on préconise de réaliser le malaxage à 80° C, pendant 5 min, à 150 tr/min.

Comme indiqué plus haut, il est recommandé que, au stade 1°, les moyens I et II soient tels que le nombre de groupes NCO libres du moyen I soit sensiblement égal au nombre de groupes OH libres du moyen II.

Au stade 2° on préconise avantageusement de poursuivre la polymérisation du PU dans la PCL à une température de l'ordre de 60° C environ à 80° C environ, pendant environ 20 min.

Dans certains cas, on pourra observer que la polymérisation du PU dans le réseau de la PCL n'est pas achevée à l'issue du stade 2°; on laissera alors ladite polymérisation se poursuivre d'elle-même lors du refroidissement qui intervient en général après le stade 2°, ou lors du stockage après démoulage.

Par ailleurs, selon la destination finale du produit, le stade 2° peut être mis en œuvre au cours d'une opération de moulage [moulage à une température de 60 à 100° C (de préférence à une température de 60 à 80° C) pendant 10 à 30 min (de préférence pendant 20 min) dans le cas de l'application en orthopédie], peut être suivi d'un moulage (également dans le cas de l'application en orthopédie), ou encore peut être suivi d'une opération d'extrusion (notamment dans le cas d'autres applications).

Les moyens I qui conviennent sont des polyisocyanates renfermant au moins 2 groupes NCO libres par molécule. Parmi ceux-ci on peut notamment mentionner (i) les di-, tri- et tétraisocyanates de formule $R(NCO)_n$ (où n est un nombre entier ayant une valeur comprise entre 2 et 4, et R est notamment un groupe aliphatique, cycloaliphatique, aryle ou aralkyle comprenant de 4 à 15 atomes de carbone) tels que les 2,4-toluènediisocyanate, 2,6-toluènediisocyanate, 4,4'-diphénylméthanediisocyanate, 1,6-hexaméthylènediisocyanate, 1,4-cyclohexanediisocyanate, 4,4'-dicyclohexylméthanediisocyanate, et isophoronediisocyanate (i.e. le diisocyanate dérivé de 2,6-diméthyl-2,5-heptadiène-4-one), (ii) les prépolymères du type polyuréthanne renfermant des groupes NCO libres et obtenus par réaction d'un polyisocyanate en excès avec un polyol, un polyoléther et/ou un polyolester, et (iii) leurs mélanges.

Parmi les moyens I mentionnés ci-dessus, les polyisocyanates les plus intéressants sont les 2,4-toluènediisocyanate, 2,6-toluènediisocyanate et 4,4'-diphénylméthanediisocyanate, les moyens préférés étant le 2,4-toluènediisocyanate et le toluènediisocyanate commercial qui renferme 80% en poids d'isomère 2,4 et 20% en poids d'isomère 2,6.

Les moyens II qui conviennent sont des polyols renfermant au moins 2 groupes OH libres par molécule. Parmi ceux-ci on peut notamment citer les polyétherpolyols ayant un poids moléculaire équivalent compris entre environ 80 et environ 400, et renfermant au moins 2 groupes OH libres par molécule. Ces polyétherpolyols sont en général obtenus par condensation d'un oxyde d'alkylène (en abrégé OA) tel que l'oxyde d'éthylène et l'oxyde de propylène avec un diol tel que l'éthylèneglycol, le propylèneglycol, le diéthylèneglycol, l'hexaméthylèneglycol, le tétraméthylèneglycol et le cyclohexyl-1,4-diméthanol, un triol, un tétraol tel que le pentaérythritol, un pentol, un hexol tel que le dulcitol et le sorbitol, et leurs mélanges. Cette condensation est réalisée en général à raison de 1 à 20 groupes OA par groupe OH libre de polyol.

Parmi les polyols II qui conviennent on peut notamment mentionner les polyesterpolyols tels que les produits commercialisés sous la nomenclature «Isonol» RMJ 101 et RMJ 104 par la Société Upjohn, et sous la nomenclature «Scuranol» P 440, P 460, P 404 et P 4004 par la Société Rhône-Poulenc.

De façon avantageuse on pourra faire appel à des polyétherpolyols disponibles dans le commerce, et en particulier aux produits fabriqués et commercialisés par la Société Pechiney-Ugine-Kuhlmann sous les nomenclatures

– Ugipol 1004, 1010, 1020, 1061, 1092 et 1093 qui sont des produits de condensation d'oxyde d'alkylène avec un ou plusieurs diols,

– Ugipol 1130, 1131, 1171, 1180, 1340, 1370, 1371 et 1372 qui sont des produits de condensation d'oxyde d'alkylène avec un ou plusieurs triols,

– Ugipol 3310, 3320, 3400, 3420, 3450, 3460 et 3602 qui sont des produits de condensation d'oxyde d'alkylène avec un ou plusieurs tétraols, pentols et/ou hexols.

Le produit obtenu au stade 2°, qui renferme

– A) 60 à 100 parties en poids de PCL,

– B) 2 à 40 parties en poids de PU, et

– le cas échéant, les moyens C et/ou D,

est utile dans plusieurs applications. Il convient notamment en tant que moyen orthopédique de contention comme cela est visé dans la présente invention; il convient également en tant que (i) moyen de détection de la chaleur, (ii) moyen isolant ou de liaison, notamment dans le domaine des joints et celui des gaines protectrices de conducteurs électriques et (iii) moyen de fixation d'inserts notamment pour remplacer les chevilles, comme indiqué respectivement dans les demandes de brevets françaises N° 82-00856, N° 82-00857 et N° 82-00858, déposées le même jour que la présente invention.

Voyons à présent l'application du produit selon l'invention en tant que moyen orthopédique de contention. Le produit est obtenu selon trois variantes $A_1$ et $B_1$ (en discontinu) et $C_1$ (en continu):

*Variante A*: après le stade 1° on arrête le malaxage, met en œuvre le stade 2° dans le malaxeur, puis moule ou lamine le produit résultant;

*Variante B*: après le stade 1° on met en œuvre le stade 2° dans un moule; et

*Variante C*: on réalise en continu le stade 1° dans un malaxeur étanche, puis en continu on injecte le mélange résultant dans des moules où l'on met en œuvre le stade 2°.

On obtient un produit (notamment sous forme de disque ou feuille) ayant une épaisseur comprise

entre environ 1 mm et environ 7 mm, et de préférence entre 2,5 mm et 4,5 mm.

Pour l'application en tant que moyen orthopédique de contention, on préconise avantageusement de mettre en œuvre le stade 1° de telle façon que l'on ait une composition finale renfermant

A) — 60 à 95 parties en poids de PCL, et

B) — 5 à 40 parties en poids de PU,

en association, le cas échéant, avec les moyens C et/ou D. On disposera ainsi d'un produit thermoplastique final qui peut être facilement formé et reformé à chaud (donc réutilisable), qui est doué d'une mémoire élastique, qui est rigide à la température ambiante et/ou à la température du corps, qui se ramollit à partir de 55° C tout en gardant une partie de sa résistance mécanique à 60° C, et qui possède l'avantage de ne pas être opaque pour les rayons X.

D'autres avantages et caractéristiques de l'invention seront mieux compris à la lecture qui va suivre d'exemples de préparation nullement limitatifs mais donnés à titre d'illustration.

*Exemple 1*

a) Dans un malaxeur étanche à pales dans lequel on maintient une température de 80° C et une agitation de 150 tr/min, on introduit successivement

— 800 g de polycaprolactone (produit commercialisé par la Société Union Carbide sous la nomenclature de «PCL 700» et ayant un poids moléculaire moyen de 40 000 environ);

— lorsque la polycaprolactone est ramollie (c'est-à-dire environ 2 à 5 min après l'introduction de la PCL), 70 g de polyétherpolyol (produit commercialisé par la Société Pechiney-Ugine-Kuhlmann sous la nomenclature de «Ugipol 3602» et ayant un poids moléculaire équivalent de 140 environ), puis

— lorsque le mélange résultant est ramolli et homogène (c'est-à-dire environ 2 à 5 min après l'introduction du polyétherpolyol), la quantité stœchiométrique (43 g) de 2,4-toluènediisocyanate.

On maintient le mélange résultant à 80° C sous agitation (150 tr/min) pendant 10 min.

b) On stoppe l'agitation et on laisse reposer le mélange ainsi obtenu dans le malaxeur à 80° C pendant 10 min.

c) On verse dans un moule rectangulaire le mélange ainsi obtenu et presse à 80° C pendant 10 min. On obtient une feuille ayant une épaisseur comprise entre 2 et 4 mm qu'on laisse refroidir à la température ambiante (15-20° C).

*Exemple 2*

a) On procède comme indiqué à l'exemple 1 a) avec un malaxeur étanche à vis en remplaçant le 2,4-toluènediisocyanate par le toluènediisocyanate commercial qui comprend 80% en poids d'isomère 2,4 et 20% en poids d'isomère 2,6.

b) On injecte en continu le mélange ainsi obtenu dans des moules, et presse à 80° C pendant 20 min.

c) On refroidit les moules jusqu'à la température ambiante, et obtient de chaque moule une feuille ayant une épaisseur de 3 à 3,5 mm.

*Exemple 3*

a) On procède comme indiqué à l'exemple 1 a) à partir de 700 g de PCL («PCL 700»), de 25 g de polyétherpolyol («Ugipol 3602») et de 15 g de toluènediisocyanate commercial.

b) On verse le mélange ainsi obtenu dans un moule et le passe à 75° C pendant 20 min.

c) On refroidit le moule jusqu'à la température ambiante pour obtenir une feuille ayant une épaisseur de 3 mm.

*Exemple 4*

a) Dans un malaxeur étanche à pales dans lequel on maintient une température de 75° C et une agitation de 200 tr/min, on introduit successivement:

— 1000 g de polycaprolactone (ayant un poids moléculaire moyen de 35 000 environ);

— lorsque la PCL est ramollie, 100 g de polyétherpolyol (50 g d'«Ugipol 3602» et 50 g d'«Ugipol 1004»), puis

— lorsque le mélange résultant est ramolli et homogène, 50 g de toluènediisocyanate commercial.

On maintient la température à 75° C et l'agitation à 200 tr/min pendant 8 min.

b) On stoppe l'agitation et on laisse la réaction de polymérisation du PU se développer pendant 10 min à 80° C.

c) On verse le mélange résultant dans un moule et presse à 60° C pendant 10 min. On obtient une feuille de 4 mm d'épaisseur.

*Exemple 5*

En procédant comme indiqué à l'exemple 2 à partir des quantités requises de PCL, d'Ugipol 3602 et de 4,4'-diphénylméthanediisocyanate, on obtient une feuille de 3 à 4 mm d'épaisseur renfermant 80 parties en poids de PCL et 20 parties en poids de PU.

*Exemple 6*

Dans un malaxeur à pales dans lequel on maintient, sous atmosphère d'azote, une agitation de 180 tr/min on prépare un prépolymérisat de moyens I et II par réaction à 110-130° C, pendant 2 à 5 min, de 100 parties en poids de «Teracol 1000» [mélange de polytétraméthylène-étherglycols de formule $HO-(CH_2-CH_2-CH_2-CH_2-O)_n-H$ où n est un nombre compris entre 6 et 42 et a pour valeur moyenne 13,63, fabriqué par la Société DuPont de Nemours] préalablement chauffé à 100-105° C pendant au moins 1 h sous vide pour éliminer les traces d'eau, avec 53 parties en poids de 4,4'-diphénylméthanediisocyanate préalablement chauffé à 70° C.

Dans le mélange réactionnel ainsi obtenu on introduit à 75° C sous agitation et sous atmosphère d'azote 13,4 parties en poids d'acide stéarique, puis 502,5 parties en poids de PCL ayant un poids moléculaire de 40 000 environ, et enfin 14,4 parties en poids de 1,4-cyclohexyldiméthanol. On laisse sous agitation le mélange ainsi obtenu pendant 5 min à 75° C pour poursuivre la réaction de polymérisation du PU.

On coule ensuite le mélange résultant dans des moules rectangulaires et presse à 100° C pendant 20 min pour obtenir, après refroidissement à 15-20° C, des feuilles ayant une épaisseur de 3 à 3,5 mm.

Dans ces conditions opératoires où les 1,4-cyclohexyldiméthanol, Teracol 1000 et 4,4'-diphénylméthanediisocyanate sont dans un rapport molaire d'environ (1:1:2), on obtient un produit final en feuille renfermant approximativement 2 parties en poids d'acide stéarique, et 25 parties en poids de PU imbriqué dans le réseau de 75 parties en poids de PCL.

*Exemple 7*

En procédant comme indiqué à l'exemple 6 à partir de 100 parties en poids de «Teracol 1000», de 53 parties en poids de 4,4'-diphénylméthanediisocyanate, de 19,4 parties en poids d'acide stéarique, de 805 parties en poids de PCL de poids moléculaire de 40000 environ, et de 11,8 parties en poids de 1,6-hexanediol on obtient, après moulage, des feuilles de 3 à 3,5 mm d'épaisseur, le produit final en feuille renfermant approximativement 2 parties en poids d'acide stéarique, et 17 parties en poids de PU imbriqué dans le réseau de 83 parties en poids de PCL.

Les feuilles selon l'invention, et en particulier celles des exemples 1 à 5, se prêtent aisément au moulage sur la portion de la surface du corps qui a besoin d'un moyen orthopédique de contention, sans qu'il soit nécessaire de forcer pour les étirer. On les réchauffe à 60° C, notamment par immersion dans de l'eau à cette température avant de les appliquer sur la peau. A chaud, elles sont autocollantes et adhèrent bien les unes aux autres sur simple pression. Après refroidissement, elles présentent une très bonne résistance au décollement, une grande rigidité, la découpe se faisant alors aux ciseaux. Après manipulation on constate qu'elles ne présentent aucune trace des doigts du manipulateur, et après application sur une portion de la peau puis découpage pour être enlevées on observe sur la surface intérieure de l'attelle les empreintes de la peau (pores, lignes de la main, etc.); ces constatations et observations montrent que les feuilles selon l'invention, du fait de leur élasticité, évitent par une légère rétraction tout flottement de l'attelle sans occasionner de pression excessive sur la surface à mouler.

Dans les autres applications décrites dans les demandes de brevets français susvisées, on utilisera avantageusement des produits obtenus au stade 2° selon l'invention renfermant:

(i) *pour l'application en tant que moyen de détection de chaleur:*

A) 80 à 95 parties en poids de PCL, et

B) 5 à 20 parties en poids de PU;

(ii) *pour l'application notamment en tant que moyen isolant ou de liaison (joints et gaines protectrices):*

A) 100 parties en poids de PCL, et

B) 5 à 50 parties en poids de PU;

(iii) *pour l'application en tant que moyen de fixation (inserts):*

A) 60 à 100 parties en poids de PCL, et

B) 5 à 40 parties en poids de PU.

## Revendications

1. Procédé pour la préparation d'une composition thermoplastique à mémoire élastique, caractérisé en ce qu'on réalise la synthèse d'un polyuréthanne, à partir des éléments connus de préparation dudit polyuréthanne à savoir au moins un polyol et au moins un polyisocyanate, au sein d'une polycaprolactone.

2. Procédé selon la revendication 1, caractérisé en ce que la formation du polyuréthanne est réalisée à partir d'un moyen I choisi parmi l'ensemble constitué par les polyisocyanates renfermant au moins deux groupes NCO libres par molécule et leurs mélanges, et d'un moyen II choisi parmi l'ensemble constitué par les polyols renfermant au moins deux groupes OH libres par molécule et leur mélanges, les moyens I et II étant tels que, avant réaction de polymérisation, le nombre de groupes NCO libres du moyen I soit approximativement égal au nombre de groupes OH libres du moyen II.

3. Procédé selon la revendication 2, caractérisé en ce que le moyen I est choisi parmi l'ensemble constitué par le 2,4-toluènediisocyanate, le 2,6-toluènediisocyanate, le 4,4'-diphénylméthanediisocyanate, le 1,6-hexaméthylènediisocyanate, le 1,4-cyclohexanediisocyanate, le 4,4'-dicyclohexylméthanediisocyanate, l'isophoronediisocyanate et leurs mélanges.

4. Procédé selon la revendication 2, caractérisé en ce que le moyen II est choisi parmi l'ensemble des polyétherpolyols obtenus par condensation d'un oxyde d'alkylène, tel que notamment l'oxyde d'éthylène et l'oxyde de propylène, avec un diol tel que l'éthylèneglycol, le propylèneglycol, le diéthylèneglycol, l'hexaméthylèneglycol, le tétraméthylèneglycol et le cyclohexyl-1,4-diméthanol, un triol, un tétraol tel que le pentaérythritol, un pentol ou un hexol tel que le dulcitol et le sorbitol, cette condensation étant réalisée à raison d'environ 1 à environ 20 groupes époxyde par groupe OH libre.

5. Procédé selon la revendication 2, caractérisé en ce que le moyen II est un polyétherpolyol de formule générale

$$HO-(CH_2-CH_2-CH_2-CH_2-O)_n-H$$

où n est un nombre compris entre 6 et 42.

6. Procédé selon l'une quelconque des revendications 1 à 5, caractérisé en ce que l'on fait réagir les moyens I et II dans la polycaprolactone de telle façon que l'on obtienne une composition finale comprenant

A) 60 à 100 parties en poids de polycaprolactone, et

B) 2 à 40 parties en poids de polyuréthanne, dans laquelle le polyuréthanne, au cours de sa polymérisation, a été imbriqué dans le réseau de la polycaprolactone.

7. Procédé selon la revendication 6, caractérisé en ce que ladite synthèse du polyuréthanne est réalisée en présence d'au moins un additif connu tel que charges minérales, colorant et/ou plastifiant.

8. Procédé selon la revendication 7, caractérisé en ce que l'on utilise au plus 35 parties en poids et de préférence 5 à 35 parties en poids de charge minérale, pour 60 à 100 parties en poids de polycaprolactone.

9. Procédé selon la revendication 7, caractérisé en ce que l'on utilise au plus 5 parties en poids et de préférence 1 à 2 parties en poids de plastifiant, pour 60 à 100 parties en poids de polycaprolactone.

10. Procédé selon l'une quelconque des revendications 1, 2 ou 6, caractérisé en ce qu'il comprend

1° le mélange sous agitation de la polycaprolactone avec les moyens I et II, à une température comprise entre 75° et 130° C pendant 1 à 10 min (de préférence pendant 5 min) pour initier la formation du polyuréthanne dans le réseau de la polycaprolactone, puis

2° la poursuite de la polymérisation du polyuréthanne dans le réseau de la polycaprolactone à une température supérieure ou égale à 60° C (et de préférence comprise entre 60° et 100° C), pendant 10 à 30 min.

11. Procédé selon l'une quelconque des revendications 1, 2 ou 6, caractérisé en ce qu'il comprend

1° le mélange sous agitation de la polycaprolactone avec le mélange réactionnel résultant de la prépolymérisation des moyens I et II, à une température comprise entre 75° et 130° C pendant 1 à 10 min (de préférence pendant 5 min) pour initier la formation du polyuréthanne dans le réseau de la polycaprolactone, puis

2° la poursuite de la polymérisation du polyuréthanne dans le réseau de la polycaprolactone à une température supérieure ou égale à 60° C (et de préférence comprise entre 60° et 100° C), pendant 10 à 30 min.

12. Procédé selon l'une quelconque des revendications 10 ou 11, caractérisé en ce que le stade 1° est mis en œuvre sous agitation à une vitesse angulaire de 50 à 300 tr/min.

13. Procédé selon l'une quelconque des revendications 10 ou 11, caractérisé en ce que le stade 1° est mis en œuvre sous agitation à vitesse angulaire de 150 tr/min, à une température de 80° C, pendant 5 min.

14. Procédé selon l'une quelconque des revendications 10 ou 11, caractérisé en ce que l'on effectue un moulage de la composition résultante après le stade 2°.

15. Procédé selon l'une quelconque des revendications 10 ou 11, caractérisé en ce que l'on effectue un moulage de la composition résultante pendant le stade 2°.

16. Produit thermoplastique obtenu selon le procédé de l'une quelconque des revendications 1 à 13, caractérisé en ce qu'il comprend

A) 60 à 100 parties en poids de polycaprolactone, et

B) 2 à 40 parties en poids de polyuréthanne en association, le cas échéant, avec un ou plusieurs moyens selon les revendications 7 à 9, le polyuréthanne ayant été au cours de sa formation imbriqué dans le réseau de la polycaprolactone.

17. Produit selon la revendication 16, caractérisé en ce qu'il comprend

A) 60 à 95 parties en poids de polycaprolactone, et

B) 5 à 40 parties en poids de polyuréthanne.

18. Utilisation du produit selon la revendication 17 en tant que moyen orthopédique de contention, caractérisé en ce que ledit produit a été soumis à un moulage à chaud selon l'une quelconque des revendications 14 ou 15, à une température de 60° à 100° C, de préférence à une température de 60° à 80° C, pendant 10 à 30 min, de préférence pendant 10 min.

## Patentansprüche

1. Verfahren zur Herstellung einer thermoplastischen Zusammensetzung mit elastischem Gedächtnis, dadurch gekennzeichnet, dass man die Synthese eines Polyurethans, ausgehend von für die Herstellung des Polyurethans bekannten Elementen, nämlich mindestens einem Polyol und mindestens einem Polyisocyanat, in einem Polycaprolacton vornimmt.

2. Verfahren nach Anspruch 1, dadurch gekennzeichnet, dass die Bildung des Polyurethans ausgehend von einem Mittel I, ausgewählt aus der Gruppe bestehend aus mindestens zwei freie NCO-Gruppen pro Molekül enthaltenden Polyisocyanaten und Mischungen davon, und einem Mittel II, ausgewählt aus der Gruppe bestehend aus mindestens zwei freie OH-Gruppen pro Molekül enthaltenden Polyolen und Mischungen davon, vorgenommen wird, wobei die Mittel I und II derart gewählt sind, dass die Anzahl an freien NCO-Gruppen des Mittels I vor der Polymerisationsreaktion annähernd gleich der Anzahl an freien OH-Gruppen des Mittels II ist.

3. Verfahren nach Anspruch 2, dadurch gekennzeichnet, dass das Mittel I ausgewählt ist aus der Gruppe bestehend aus 2,4-Toluoldiisocyanat, 2,6-Toluoldiisocyanat, 4,4'-Diphenylmethandiisocyanat, 1,6-Hexamethylendiisocyanat, 1,4-Cyclohexandiisocyanat, 4,4'-Dicyclohexylmethandiisocyanat, Isophorondiisocyanat und Mischungen davon.

4. Verfahren nach Anspruch 2, dadurch gekennzeichnet, dass das Mittel II ausgewählt ist aus der Gruppe bestehend aus Polyätherpolyolen, die erhalten wurden durch Kondensation eines Alkylenoxids, wie insbesondere Äthylenoxid und Propylenoxid, mit einem Diol, wie Äthylenglykol, Propylenglykol, Diäthylenglykol, Hexamethylenglykol, Tetramethylenglykol und Cyclohexyl-1,4-dimethanol, einem Triol, einem Tetraol, wie Pentaerythrit, einem Pentol oder einem Hexol, wie Dulcit und Sorbit, welche Kondensation in einer Menge von etwa 1 bis etwa 20 Epoxidgruppen pro freie OH-Gruppe durchgeführt wird.

5. Verfahren nach Anspruch 2, dadurch gekennzeichnet, dass das Mittel II ein Polyätherpolyol der allgemeinen Formel

$$HO-(CH_2-CH_2-CH_2-CH_2-O)_n-H$$

ist, worin n eine Zahl zwischen 6 und 42 bedeutet.

6. Verfahren nach einem der Ansprüche 1 bis 5, dadurch gekennzeichnet, dass man die Mittel I und II in Polycaprolacton derart zur Umsetzung bringt, dass man eine Endzusammensetzung erhält, die

A) 60 bis 100 Gewichtsteile Polycaprolacton und

B) 2 bis 40 Gewichsteile Polyurethan

enthält, wobei das Polyurethan im Verlauf seiner Polymerisation in das Polycaprolactongitter eingebaut wird.

7. Verfahren nach Anspruch 6, dadurch gekennzeichnet, dass die Polyurethansynthese in Gegenwart von zumindest einem bekannten Zusatzstoff, wie mineralischen Füllstoffen, Farbstoffen und/oder Plastifizierungsmitteln, durchgeführt wird.

8. Verfahren nach Anspruch 7, dadurch gekennzeichnet, dass man höchsten 35 Gewichtsteile, vorzugsweise 5 bis 35 Gewichtsteile mineralischen Füllstoff pro 60 bis 100 Gewichsteile Polycaprolacton verwendet.

9. Verfahren nach Anspruch 7, dadurch gekennzeichnet, dass man höchstens 5 Gewichtsteile, vorzugsweise 1 bis 2 Gewichtsteile Plastifizierungsmittel pro 60 bis 100 Gewichtsteile Polycaprolacton verwendet.

10. Verfahren nach einem der Ansprüche 1, 2 oder 6, dadurch gekennzeichnet, dass es folgende Schritte umfasst:

1. Mischung von Polycaprolacton mit den Mitteln I und II unter Rühren bei einer Temperatur zwischen 75° und 130° C während 1 bis 10 min (vorzugsweise 5 min) zur Einleitung der Bildung von Polyurethan im Polycaprolactongitter und danach

2. Fortsetzung der Polymerisation von Polyurethan im Polycaprolactongitter bei einer Temperatur von über oder gleich 60° C (vorzugsweise zwischen 60° und 100° C) während 10 bis 30 min.

11. Verfahren nach einem der Ansprüche 1, 2 oder 6, dadurch gekennzeichnet, dass es folgende Schritte umfasst:

1. Mischung von Polycaprolacton mit der aus der Vorpolymerisation der Mittel I und II stammenden Reaktionsmischung bei einer Temperatur zwischen 75° und 130° C während 1 bis 10 min (vorzugsweise 5 min) zur Einleitung der Bildung von Polyurethan im Polycaprolactongitter und danach

2. Fortsetzung der Polymerisation von Polyurethan im Polycaprolactongitter bei einer Temperatur von über oder gleich 60° C (vorzugsweise zwischen 60° und 100° C) während 10 bis 30 min.

12. Verfahren nach einem der Ansprüche 10 oder 11, dadurch gekennzeichnet, dass die Stufe 1 unter Rühren mit einer Winkelgeschwindigkeit von 50 bis 300 U/min durchgeführt wird.

13. Verfahren nach einem der Ansprüche 10 oder 11, dadurch gekennzeichnet, dass die Stufe 1 unter Rühren mit einer Winkelgeschwindigkeit von 150 U/min bei einer Temperatur von 80° C während 5 min durchgeführt wird.

14. Verfahren nach einem der Ansprüche 10 oder 11, dadurch gekennzeichnet, dass man eine Formung der nach der Stufe 2 resultierenden Zusammensetzung vornimmt.

15. Verfahren nach einem der Ansprüche 10 oder 11, dadurch gekennzeichnet, dass man eine Formung der während der Stufe 2 resultierenden Zusammensetzung vornimmt.

16. Thermoplastisches Material, erhalten nach dem Verfahren eines der Ansprüche 1 bis 13, dadurch gekennzeichnet, dass es folgendes umfasst:

A) 60 bis 100 Gewichtsteile Polycaprolacton und

B) 2 bis 40 Gewichtsteile Polyurethan,

gegebenenfalls in Verbindung mit einem oder mehreren Mitteln nach den Ansprüchen 7 bis 9, wobei das Polyurethan im Verlauf seiner Bildung in das Polycaprolactongitter eingebaut worden ist.

17. Produkt nach Anspruch 16, dadurch gekennzeichnet, dass es folgendes umfasst:

A) 60 bis 95 Gewichtsteile Polycaprolacton und

B) 5 bis 40 Gewichtsteile Polyurethan.

18. Verwendung des Produkts nach Anspruch 17 als orthopädisches Kontentivmittel, dadurch gekennzeichnet, dass das Produkt einer Wärmeformung nach einem der Ansprüche oder 15 bei einer Temperatur von 60° bis 100° C, vorzugsweise 60° bis 80° C, während 10 bis 30 min, vorzugsweise 10 min, unterzogen wurde.

## Claims

1. Process for the preparation of a thermoplastic composition with elastic memory, characterized in that the formation of polyurethane is carried out from known elements of preparation of polyurethane, namely at least one polyol and at least one polyisocyanate, within the polycaprolactone.

2. Process according to claim 1, characterized in that the formation of polyurethane is carried out from a means I selected from among the group constituted by polyisocyanates containing at least two free NCO groups per molecule and their mixtures, and a means II selected from among the group constituted by polyols containing at least two free OH groups per molecule and their mixtures, the means I and II being such that prior to the polymerization reaction, the number of free NCO groups of the means I is approximately equal to the number of free OH groups of means II.

3. Process according to claim 2, characterized in that means I is selected from among the group constituted by 2,4-toluenediisocyanate, 2,6-toluenediisocyanate, 4,4'-diphenylmethanediisocyanate, 1,6 hexamethylenediisocyanate, 1,4-cyclohexanediisocyanate, 4,4'-dicyclohexyl-

methanediisocyanate, isophoronediisocyanate and their mixtures.

4. Process according to claim 2, characterized in that means II is selected from among the group of polyetherpolyols obtained by condensation of an alkylene oxide such as particularly ethylene oxide and propylene oxide, with a diol such as ethyleneglycol, propyleneglycol, diethyleneglycol, hexamethyleneglycol, tetramethyleneglycol and cyclohexyl-1,4-dimethanol, a triol, a tetraol such as pentaerythritol, a pentol or a hexol such as dulcitol and sorbitol, this condensation being carried out in the proportion of about 1 to about 20 epoxide groups per free OH group.

5. Process according to claim 2, characterized in that means II is a polyetherpolyol of the general formula

$$HO-(CH_2-CH_2-CH_2-CH_2-O)_n-H$$

where n is a number comprised between 6 and 42.

6. Process according to any one of claims 1 to 5, characterized in that means I and II are reacted in the polycaprolactone so that a final composition is obtained comprising

A) 60 to 100 parts by weight of polycaprolactone, and

B) 2 to 40 parts by weight of polyurethane,

in which the polyurethane, in the course of its polymerization, has been imbricated in the network of the polycaprolactone.

7. Process according to claim 6, characterized in that the formation of the polyurethane in the polycaprolactone is carried out in the presence of at least one known additive such as inorganic fillers, colouring agents and plasticizers.

8. Process according to claim 7, characterized in that there are used at the most 35 parts by weight and preferably 5 to 35 parts by weight of inorganic filler, per 60 to 100 parts by weight of polycaprolactone.

9. Process according to claim 7, characterized in that there are used at the most 5 parts by weight and preferably 1 to 2 parts by weight of plasticizer, per 60 to 100 parts by weight of polycaprolactone.

10. Process according to any one of claims 1, 2 or 6, characterized in that it comprises

1. mixing with stirring polycaprolactone with means I and II, at a temperature comprised between 75° and 130° C for 1 to 10 min (preferably for 5 min) to initiate formation of the polyurethane in the network of the polycaprolactone, then

2. continuing the polymerization of the polyurethane in the network of the polycaprolactone at a temperature higher than or equal to 60° C (and preferably comprised between 60° and 100° C), for 10 to 30 min.

11. Process according to any one of claims 1, 2 or 6, characterized in that it comprises:

1. the mixing with stirring of the polycaprolactone with the reaction mixture resulting from the prepolymerization of means I and II, at a temperature comprised between 75° and 130° C for 1 to 10 min (preferably for 5 min) to initiate the formation of the polyurethane in the network of the polycaprolactone, then

2. continuing the polymerization of the polyurethane in the network of the polycaprolactone at a temperature above or equal to 60° C (and preferably comprised between 60° and 100° C), for 10 to 30 min.

12. Process according to any one of claims 10 or 11, characterized in that stage 1 is employed with stirring at an angular speed of 50 to 300 r.p.m.

13. Process according to any one of claims 10 or 11, characterized in that stage 1 is applied with stirring at an angular speed of 150 r.p.m., at a temperature of 80° C, for 5 min.

14. Process according to any one of claims 10 or 11, characterized in that moulding of the resulting composition is carried out after stage 2.

15. Process according to any one of claims 10 or 11, characterized in that moulding of the resulting composition is carried out during stage 2.

16. Thermoplastic product obtained according to the process of any one of claims 1 to 13, characterized in that it comprises:

A) 60 to 100 parts by weight of polycaprolactone, and

B) 2 to 40 parts by weight of polyurethane

in association, if necessary, with one or several means according to claims 7 to 9, the polyurethane having been in the course of its formation imbricated in the network of the polycaprolactone.

17. Product according to claim 16, characterized in that it comprises:

A) 60 to 95 parts by weight of polycaprolactone, and

B) 5 to 40 parts by weight of polyurethane.

18. Use of the product according to claim 17 as orthopedic immobilising means, characterized in that said product has been subjected to hot moulding according to any one of claims 14 or 15, at a temperature of 60° to 100° C, preferably at a temperature of 60° to 80° C, for 10 to 30 min, preferably for 10 min.